# EUROPEAN PATENT SPECIFICATION

(11) **EP 1 966 184 B1**
(45) Date of publication and mention of the grant of the patent: **25.08.2010**
(21) Application number: 06841392.1
(22) Date of filing: 18.12.2006
(51) Int. Cl.: C07D 401/04, A61K 31/517, A61P 11/06

(54) **PYRIDINYL-QUINAZOLINE DERIVATIVES AND THEIR MEDICAL USE**
PYRIDINYLCHINAZOLINDERIVATE UND IHRE MEDIZINISCHE VERWENDUNG
DERIVES DE PYRIDINYL-QUINAZOLINE ET LEUR UTILISATION MEDICALE

(30) Priority: 20.12.2005 DK 200501801; 21.12.2005 US 752036 P
(43) Date of publication of application: 10.09.2008
(73) Proprietor: NeuroSearch A/S, 2750 Ballerup (DK)
(72) Inventor: ERIKSEN, Birgitte, L., DK-2750 Ballerup (DK); SØRENSEN, Ulrik, Svane, DK-2750 Ballerup (DK); TEUBER, Lene, DK-3500 Værløse (DK); PETERS, Dan, DK-2750 Ballerup (DK); HOUGAARD, Charlotte, DK-2750 Ballerup (DK); JOHANSEN, Tina, Holm, DK-2765 Smørum (DK); CHRISTOPHERSEN, Palle, DK-2750 Ballerup (DK)
(74) Representative: Madsen, Inger Margrethe Schelde
(86) International application number: PCT/EP2006/069795
(87) International publication number: WO 2007/071632

(56) References cited:
- EP-A1- 0 579 496
- EP-A2- 0 135 975
- WO-A-2006/071095
- JP-A- 8 003 144
- LEE, SUNG J. ET AL: "Discovery of Potent cGMP-PDE Inhibitors" JOURNAL OF MEDICINAL CHEMISTRY, vol. 38, no. 18, 1995, pages 3547-3557, XP002438103
- DATABASE CHEMCATS STN; 18 January 2005 (2005-01-18), XP002438106 Database accession no. 2006:5006117 CHEMCATS & DATABASE REGISTRY STN; 18 January 2005 (2005-01-18),
- DATABASE CHEMCATS STN; 18 January 2005 (2005-01-18), XP002438571 Database accession no. 2006:5008621 CHEMCATS & DATABASE REGISTRY STN; 18 January 2005 (2005-01-18),

## Description

### TECHNICAL FIELD

This invention relates to novel pyridinyl-quinazoline derivatives and their use as potassium channel modulating agents. Moreover the invention is directed to pharmaceutical compositions useful for the treatment or alleviation of diseases or disorders associated with the activity of potassium channels.

### BACKGROUND ART

Ion channels are transmembrane proteins, which catalyse the transport of inorganic ions across cell membranes. The ion channels participate in processes as diverse as the generation and timing of action potentials, synaptic transmissions, secretion of hormones, contraction of muscles, etc.

All mammalian cells express potassium (K⁺) channels in their cell membranes, and the channels play a dominant role in the regulation of the membrane potential. In nerve and muscle cells they regulate the frequency and form of the action potential, the release of neurotransmitters, and the degree of broncho- and vasodilation.

From a molecular point of view, the K⁺ channels represent the largest and most diverse group of ion channels. For an overview they can be divided into five large subfamilies: Voltage-activated K⁴ channels (Kᵥ), long QT related K⁴ channels (KvLQT), inward rectifiers (K_{IR}), two-pore K⁺ channels (K_{TP}), and calcium-activated K⁺ channels (K_{ca}).

The latter group, the Ca²+-activated K⁺ channels, consists of three well-defined subtypes: SK channels, IK channels and BK channels. SK, IK and BK refer to the single-channel conductance (Small, Intermediate and Big conductance K channel). The SK, IK, and BK channels exhibit differences in e.g. voltage- and calcium-sensitivity, pharmacology, distribution and function.

SK channels are present in many central neurons and ganglia, where their primary function is to hyperpolarize nerve cells following one or several action potentials, in order to prevent long trains of epileptogenic activity to occur. The SK channels are also present in several peripheral cells including skeletal muscle, gland cells, liver cells, and T-lymphocytes. The significance of SK channels in normal skeletal muscle is not clear, but their number is significantly increased in denervated muscle, and the large number of SK channels in the muscle of patients with myotonic muscle dystrophia, suggest a role in the pathogenesis of the disease.

Studies indicate that K⁺ channels may be a therapeutic target in the treatment of a number of diseases including asthma, cystic fibrosis, chronic obstructive pulmonary disease and rhinorrhea, convulsions, vascular spasms, coronary artery spasms, renal disorders, polycystic kidney disease, bladder spasms, urinary incontinence, bladder outflow obstruction, irritable bowel syndrome, gastrointestinal dysfunction, secretory diarrhoea, ischaemia, cerebral ischaemia, ischaemic heart disease, angina pectoris, coronary heart disease, traumatic brain injury, psychosis, anxiety, depression, dementia, memory and attention deficits, Alzheimer's disease, dysmenorrhea, narcolepsy, Reynaud's disease, intermittent claudication, Sjorgren's syndrome, migraine, arrhythmia, hypertension, absence seizures, myotonic muscle dystrophia, xerostomi, diabetes type II, hyperinsulinemia, premature labour, baldness, cancer and immune suppression.

WO 2005/082884 discloses sulfanyl-pyridinyl-pyrimidine compounds useful as mGluR antagonists. However, the pyridinyl-quinazoline derivatives of the present invention are not disclosed.

EP 579496 describes a very large group of 4-aminoquinazoline derivatives and their use as medicine.

Sung J. Lee et al., J. Med. Chem. 1995 38 pp 3547-3557,Discovery of potent cyclic GMP phosphodiesterase inhibitors, describes 2-pyridyl- and 2-imidazolylquinazolines possessing cyclic GMP phosphodiesterase and thromboxane synthesis inhibitory activities.

DATABASE CHEMCATS 2006:5006117 (18 Jan 2005) and DATABASE CHEMCATS 2006:5008621 (18 Jan 2005) describe compounds, which comprises unsubstituted 2-pyridyl groups.

WO 2006/071095 describes a very large group of quinazoline derivatives for the treatment of diabetes and obesity.

### SUMMARY OF THE INVENTION

The present invention resides in the provision of novel chemical compounds capable of modulating SK channels, or subtypes of SK channels.

Accordingly, in its first aspect, the invention provides novel pyridinyl-quinazoline derivative of Formula I 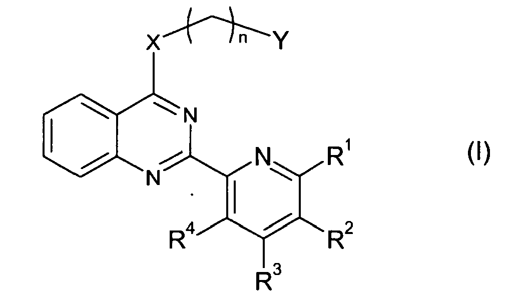 an enantiomer or a mixture of its enantiomers, an N-oxide thereof, or a pharmaceutically acceptable salt thereof, wherein
n is 0, 1, 2 or 3;
X represents O, S or NR'; wherein
R' represents hydrogen, alkyl, cycloalkyl or cycloalkyl-alkyl;
or, when n is 0 and X is NR', R' together with Y and together with the nitrogen to which they are attached form a heterocyclic ring, which heterocyclic ring may optionally be substituted with alkyl of phenyl;
Y represents alkyl, cycloalkyl, alkyl-cycloalkyl, alkenyl or phenyl, which phenyl is optionally substituted one or more times with substituents selected from the group consisting of alkyl, amino-alkyl, alkyl-amino, alkyl-amino-alkyl, hydroxy-alkyl, alkoxy-alkyl, cycloalkyl, cycloalkyl-alkyl, alkenyl, halo, haloalkyl, hydroxy, alkoxy, haloalkoxy, cyano, nitro and amino;
or, when n is 0, Y together with R' and together with the nitrogen to which they are attached form a heterocyclic ring, which heterocyclic ring may optionally be substituted with alkyl of phenyl; and
R¹ represents methyl, ethyl, propyl, fluoro, chloro or bromo; and R², R³ and R⁴ represent hydrogen, or
R¹, R² and R³ represent hydrogen; and R⁴ represent benzoyl, or
R¹ and R², or R² and R³, or R³ and R⁴, respectively, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and
the remaining of R¹, R², R³ and R⁴ represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl.

In another aspect, the invention provides pharmaceutical compositions comprising an effective amount of a chemical compound of the invention.

In further aspects the invention relates to the use of a chemical compound of the invention for the manufacture of a medicament for the treatment or alleviation of diseases or disorders associated with the activity of potassium channels.

### DETAILED DISCLOSURE OF THE INVENTION

### Potassium Channel Modulating Agents

In its first aspect, the invention provides novel pyridinyl-quinazoline derivative of Formula I 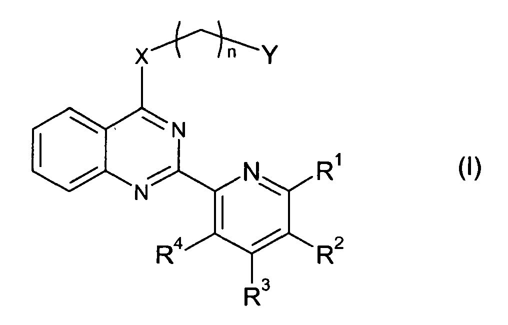 an enantiomer or a mixture of its enantiomers, an N-oxide thereof, or a pharmaceutically acceptable salt thereof, wherein
n is 0, 1, 2 or 3;
X represents O, S or NR'; wherein
R' represents hydrogen, alkyl, cycloalkyl or cycloalkyl-alkyl;
or, when n is 0 and X is NR', R' together with Y and together with the nitrogen to which they are attached form a heterocyclic ring, which heterocyclic ring may optionally be substituted with alkyl of phenyl;
Y represents alkyl, cycloalkyl, alkyl-cycloalkyl, alkenyl or phenyl, which phenyl is optionally substituted one or more times with substituents selected from the group consisting of alkyl, amino-alkyl, alkyl-amino, alkyl-amino-alkyl, hydroxy-alkyl, alkoxy-alkyl, cycloalkyl, cycloalkyl-alkyl, alkenyl, halo, haloalkyl, hydroxy, alkoxy, haloalkoxy, cyano, nitro and amino;
or, when n is 0, Y together with R' and together with the nitrogen to which they are attached form a heterocyclic ring, which heterocyclic ring may optionally be substituted with alkyl of phenyl; and
R¹ represents methyl, ethyl, propyl, fluoro, chloro or bromo; and R², R³ and R⁴ represent hydrogen, or
R¹, R² and R³ represent hydrogen; and R⁴ represent benzoyl, or
R¹ and R², or R² and R³, or R³ and R⁴, respectively, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and
the remaining of R¹, R², R³ and R⁴ represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl;
*provided,* however, that the pyrazolyl-quinoline derivative is not Cyclohexyl-(2-pyridin-2-yl-quinazolin-4-yl)-amine]; 2-Pyridin-2-yl-4-p-tolyloxy-quinazoline; (4-Fluoro-phenyl)-(2-pyridin-2-yl-quinazolin-4-yl)-amine; (2-Pyridin-2-yl-quinazolin-4-yl)-p-tolyl-amine; 4-Phenyl-2-pyridin-2-yl-quinazoline; Benzyl-(2-pyridin-2-yl-quinazolin-4-yl)-amine; Phenethyl-(2-pyridin-2-yl-quinazolin-4-yl)-amine;

In a preferred embodiment pyridinyl-quinazoline derivative of the invention is a compound of Formula I wherein n is 0, 1, 2 or 3.

In a more preferred embodiment n is 0, 1 or 2.

In an even more preferred embodiment n is 0 or 1.

In a most preferred embodiment n is 0.

In another preferred embodiment pyridinyl-quinazoline derivative of the invention is a compound of Formula I wherein X represents O, S or NR'; wherein R' represents hydrogen, alkyl, cycloalkyl or cycloalkyl-alkyl; or, when n is 0 and X is NR', R' together with Y and together with the nitrogen to which they are attached form a heterocyclic ring, which heterocyclic ring may optionally be substituted with alkyl of phenyl.

In a more preferred embodiment X represents NR'; wherein R' represents hydrogen, alkyl, cycloalkyl or cycloalkyl-alkyl.

In a most preferred embodiment X represents NH.

In a third preferred embodiment pyridinyl-quinazoline derivative of the invention is a compound of Formula I wherein Y represents alkyl, cycloalkyl, alkyl-cycloalkyl, alkenyl or phenyl, which phenyl is optionally substituted one or more times with substituents selected from the group consisting of alkyl, amino-alkyl, alkyl-amino, alkyl-amino-alkyl, hydroxy-alkyl, alkoxy-alkyl, cycloalkyl, cycloalkyl-alkyl, alkenyl, halo, haloalkyl, hydroxy, alkoxy, haloalkoxy, cyano, nitro and amino; or, when n is 0, Y together with R' and together with the nitrogen to which they are attached form a heterocyclic ring, which heterocyclic ring may optionally be substituted with alkyl of phenyl.

In a more preferred embodiment Y represents alkyl, cycloalkyl, alkyl-cycloalkyl, alkenyl or phenyl, which phenyl is optionally substituted one or more times with substituents selected from the group consisting of alkyl, amino-alkyl, alkyl-amino, alkyl-amino-alkyl, hydroxy-alkyl, alkoxy-alkyl, cycloalkyl, cycloalkyl-alkyl, alkenyl, halo, haloalkyl, hydroxy, alkoxy, haloalkoxy, cyano, nitro and amino.

In an even more preferred embodiment Y represents alkyl, cycloalkyl, alkyl-cycloalkyl, phenyl or benzyl, which phenyl or benzyl is optionally substituted with alkyl, halo or haloalkyl.

In a still more preferred embodiment Y represents cyclopentyl, cyclohexyl, cycloheptyl, phenyl or benzyl, which phenyl is optionally substituted with alkyl, halo or haloalkyl.

In a yet more preferred embodiment Y represents cyclopentyl, cyclohexyl, cycloheptyl, phenyl or benzyl, which phenyl is optionally substituted with methyl, chloro or trifluoromethyl.

In a further preferred embodiment R¹ represents methyl, ethyl, propyl, fluoro, chloro or bromo; and R², R³ and R⁴ represent hydrogen.

In a still further preferred embodiment R¹ represents methyl, ethyl, propyl, fluoro or chloro; and R², R³ and R⁴ represent hydrogen.

In a still further preferred embodiment R¹, R² and R³ represent hydrogen; and R⁴ represent benzoyl.

In a still further preferred embodiment R¹, R² and R³ represent hydrogen; and R⁴ represent benzoyl.

In a still further preferred embodiment R³ and R⁴, independently of each other, represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and R¹ and R², together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl.

In a still further preferred embodiment R¹ and R⁴, independently of each other, represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and R² and R³, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl.

In another preferred embodiment R¹ and R², independently of each other, represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and R³ and R⁴, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl

In a more preferred embodiment R¹ and R², independently of each other, represent hydrogen, alkyl, cycloalkyl, cycloalkyl-alkyl, alkenyl, halo, haloalkyl, hydroxy, alkoxy, haloalkoxy, alkoxy-carbonyl, cyano, nitro and amino; and R³ and R⁴, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro and amino.

In an even more preferred embodiment R¹ and R², independently of each other, represent hydrogen, alkyl, cycloalkyl or halo; and R³ and R⁴, together form a benzo-fused carbocyclic ring.

In a still more preferred embodiment R¹ and R² represent hydrogen; and R³ and R⁴, together form a benzo-fused carbocyclic ring.

In a fifth preferred embodiment pyridinyl-quinazoline derivative of the invention is a compound of Formula I wherein n is 0 or 1; X represents NH; Y represents cycloalkyl or phenyl, which phenyl is optionally substituted with alkyl or halo; R¹ represents methyl, ethyl, propyl, fluoro, chloro or bromo; and R², R³ and R⁴ represent hydrogen, or R¹, R² and R³ represent hydrogen; and R⁴ represent benzoyl, ; or R¹ and R² represent hydrogen; and R³ and R⁴, together form a benzo-fused carbocyclic ring.

In a more preferred embodiment n is 0; X represents NH; Y represents cycloalkyl, phenyl or benzyl, which phenyl is optionally substituted with alkyl or halo; R¹ represents methyl, ethyl, propyl, fluoro, chloro or bromo; and R², R³ and R⁴ represent hydrogen, or R¹, R² and R³ represent hydrogen; and R⁴ represent benzoyl,

In a most preferred embodiment the pyridinyl-quinazoline derivative of the invention is
(4-Chloro-phenyl)-[2-(6-methyl-pyridin-2-yl)-quinazolin-4-yl]-amine;
[2-(4-Cyclohexylamino-quinazolin-2-yl)-pyridin-3-yl]-phenyl-methanone;
Cyclohexyl-[2-(6-methyl-pyridin-2-yl)-quinazolin-4-yl]-amine;
(4-Chloro-benzyl)-[2-(6-methyl-pyridin-2-yl)-quinazolin-4-yl]-amine;
[2-(6-Chloro-pyridin-2-yl)-quinazolin-4-yl]-cyclohexyl-amine;
(4-Chloro-phenyl)-[2-(6-chloro-pyridin-2-yl)-quinazolin-4-yl]-amine;
(4-Chloro-benzyl)-[2-(6-chloro-pyridin-2-yl)-quinazolin-4-yl]-amine;
{2-[4-(4-Chloro-phenylamino)-quinazolin-2-yl]-pyridin-3-yl}-phenyl-methanone;
{2-[4-(4-Chloro-benzylamino)-quinazolin-2-yl]-pyridin-3-yl}-phenyl-methanone;
Cyclohexyl-(2-isoquinolin-1-yl-quinazolin-4-yl)-amine;
(4-Chloro-phenyl)-(2-isoquinolin-1-yl-quinazolin-4-yl)-amine;
(4-Chloro-benzyl)-(2-isoquinolin-1-yl-quinazolin-4-yl)-amine;
(4-Chloro-phenyl)-[2-(6-fluoro-pyridin-2-yl)-quinazolin-4-yl]-amine;
[2-(6-Bromo-pyridin-2-yl)-quinazolin-4-yl]-(4-chloro-phenyl)-amine;
or a pharmaceutically acceptable salt thereof.

### Definition of Substituents

In the context of this invention halo represents fluoro, chloro, bromo or iodo. Thus a trihalomethyl group represents e.g. a trifluoromethyl group, a trichloromethyl group, and similar trihalo-substituted methyl groups.

In the context of this invention a haloalkyl group designates an alkyl group as defined herein, which alkyl group is substituted one or more times with halo. Preferred haloalkyl groups of the invention include trihalomethyl, preferably trifluoromethyl.

In the context of this invention an alkyl group designates a univalent saturated, straight or branched hydrocarbon chain. The hydrocarbon chain preferably contain of from one to eighteen carbon atoms (C₁₋₁₈-alkyl), more preferred of from one to six carbon atoms (C₁₋₆-alkyl; lower alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl, and tertiary butyl. In a preferred embodiment of this invention alkyl represents a C₁₋₃-alkyl group, which may in particular be methyl, ethyl, propyl or isopropyl.

In the context of this invention an alkenyl group designates a carbon chain containing one or more double bonds, including di-enes, tri-enes and poly-enes. In a preferred embodiment the alkenyl group of the invention comprises of from two to eight carbon atoms (C₂₋₈-alkenyl), more preferred of from two to six carbon atoms (C₂₋₆ -alkenyl), including at least one double bond. In a most preferred embodiment the alkenyl group of the invention is ethenyl; 1- or 2-propenyl; 1-, 2- or 3-butenyl, or 1,3-butenyl; 1-, 2-, 3-, 4- or 5-hexenyl, or 1,3-hexenyl, or 1,3,5-hexenyl; 1-, 2-, 3-, 4-, 5-, 6-, or 7-octenyl, or 1,3-octenyl, or 1,3,5-octenyl, or 1,3,5,7-octenyl.

In the context of this invention a hydroxy-alkyl group designates an alkyl group as defined above, which hydroxy-alkyl group is substituted with one or more hydroxy groups. Examples of preferred hydroxy-alkyl groups of the invention include 2-hydroxy-ethyl, 3-hydroxy-propyl, 4-hydroxy-butyl, 5-hydroxy-pentyl and 6-hydroxyhexyl.

In the context of this invention a cycloalkyl group designates a cyclic alkyl group, preferably containing of from three to ten carbon atoms (C₃₋₁₀-cycloalkyl), preferably of from three to eight carbon atoms (C₃₋₈-cycloalkyl), including cyclopropyl, cyclobutyl, cyclopentyl, cyclohexyl, cycloheptyl and cyclooctyl.

In the context of this invention a cycloalkyl-alkyl group designates a cycloalkyl group as defined above, which cycloalkyl group is substituted on an alkyl group as also defined above. Examples of preferred cycloalkyl-alkyl groups of the invention include cyclopropylmethyl and cyclopropylethyl.

In the context of this invention an alkoxy group designates an "alkyl-O-" group, wherein alkyl is as defined above.

In the context of this invention a haloalkoxy group designates an alkoxy group as defined herein, which alkoxy group is substituted one or more times with halo. Preferred haloalkoxy groups of the invention include trihalomethoxy, preferably trifluoromethoxy.

In the context of this invention an amino group may be a primary (-NH₂), secondary (-NH-alkyl), or tertiary (-N(alkyl)₂) amino group, i.e. it may be substituted once or twice with an alkyl group as defined above.

In the context of this invention a mono- or poly-cyclic carbocyclic group designates a mono- or polycyclic hydrocarbon group, which group may in particular be an aromatic hydrocarbon group, i.e. a mono- or polycyclic aryl group, or a saturated hydrocarbon group, or a partially saturated hydrocarbon group. Preferred poly-carbocyclic group are the bicyclic carbocyclic groups.

In the context of this invention a monocyclic or polycyclic, carbocyclic group designates a monocyclic or polycyclic hydrocarbon group. Examples of preferred carbocyclic groups of the invention include cycloalkyl, phenyl, naphthyl, indenyl, azulenyl, anthracenyl, and fluorenyl. Most preferred carbocyclic groups of the invention include phenyl, naphthyl and 1,2,3,4-tetrahydro-naphthyl.

In the context of this invention a monocyclic or polycyclic, heterocyclic group designates a mono- or polycyclic group, which group holds one or more heteroatoms in its ring structure. Preferred heteroatoms include nitrogen (N), oxygen (O), and sulphur (S). One or more of the ring structures may in particular be aromatic (i.e. a heteroaryl), saturated or partially saturated. Preferred heterocyclic monocyclic groups of the invention include 5- and 6 membered heterocyclic monocyclic groups. Preferred poly-heterocyclic groups of the invention are the bicyclic heterocyclic groups.

Examples of preferred heterocyclic monocyclic groups of the invention include pyrrolidinyl, in particular pyrrolidin-1-yl, pyrrolidin-2-yl, and pyrrolidin-3-yl; piperidinyl, in particular piperidin-1-yl, piperidin-2-yl, piperidin-3-yl and piperidin-4-yl; furanyl, in particular furan-2-yl and furan-3-yl; thienyl, in particular thien-2-yl and thien-3-yl; and pyrrolyl, in particular pyrrol-1-yl, pyrrol-2-yl and pyrrol-3-yl.

### Steric Isomers

The chemical compounds of the present invention may exist in (+) and (-) forms as well as in racemic forms. The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of *d*- or *l*- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Moreover, some of the chemical compounds of the invention being oximes, may thus exist in two forms, syn- and anti-form (Z- and E-form), depending on the arrangement of the substituents around the -C=N- double bond. A chemical compound of the present invention may thus be the syn- or the anti-form (Z- and E-form), or it may be a mixture hereof.

### Pharmaceutically Acceptable Salts

The chemical compound of the invention may be provided in any form suitable for the intended administration. Suitable forms include pharmaceutically (i.e. physiologically) acceptable salts of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulfonate derived from benzensulfonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulfonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.Examples of pharmaceutically acceptable cationic salts of a chemical compound of the invention include, without limitation, the sodium, the potassium, the calcium, the magnesium, the zinc, the aluminium, the lithium, the choline, the lysine, and the ammonium salt, and the like, of a chemical compound of the invention containing an anionic group. Such cationic salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

The chemical compound of the invention may be provided in dissoluble or indissoluble forms together with a pharmaceutically acceptable solvent such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

### Methods of Preparation

The chemical compounds of the invention may be prepared by conventional methods of chemical synthesis, e.g. those described in the working examples. The starting materials for the processes described in the present application are known or may readily be prepared by conventional methods from commercially available chemicals.

The end products of the reactions described herein may be isolated by conventional techniques, e.g. by extraction, crystallisation, distillation, chromatography, etc.

### Biological Activity

The chemical compounds of the invention have been subjected to *in vitro* experiments and found particularly useful as potassium channel modulating agents. More particularly the compounds of the invention are capable of selectively modulating SK1, SK2 and/or SK3 channels.

Therefore, in another aspect, the invention relates to the use of a chemical compound of the invention for the manufacture of medicaments, which medicament may be useful for the treatment or alleviation of a disease or a disorder associated with the activity of potassium channels, in particular SK channels, more particularly SK1, SK2 and/or SK3 channels.

In a preferred embodiment, the disease or a disorder associated with the activity of potassium channels is a respiratory disease, epilepsy, convulsions, seizures, absence seizures, vascular spasms, coronary artery spasms, renal disorders, polycystic kidney disease, bladder spasms, urinary incontinence, bladder outflow obstruction, erectile dysfunction, gastrointestinal dysfunction, secretory diarrhoea, ischaemia, cerebral ischaemia, ischaemic heart disease, angina pectoris, coronary heart disease, ataxia, traumatic brain injury, Parkinson's disease, bipolar disorder, psychosis, schizophrenia, anxiety, depression, mood disorders, dementia, memory and attention deficits, Alzheimer's disease, amyotrophic lateral sclerosis (ALS), dysmenorrhea, narcolepsy, Reynaud's disease, intermittent claudication, Sjorgren's syndrome, arrhythmia, hypertension, myotonic muscle dystrophia, spasticity, xerostomi, diabetes type II, hyperinsulinemia, premature labour, baldness, cancer, irritable bowel syndrome, immune suppression, migraine or pain.

In a more preferred embodiment the disease or a disorder associated with the activity of potassium channels is a respiratory disease, urinary incontinence, erectile dysfunction, anxiety, epilepsy, psychosis, schizophrenia, amyotrophic lateral sclerosis (ALS) or pain.

In another preferred embodiment the disease or a disorder associated with the activity of potassium channels is a respiratory disease, in particular asthma, cystic fibrosis, chronic obstructive pulmonary disease (COPD) or rhinorrhea.

In a third preferred embodiment the disease or a disorder associated with the activity of potassium channels is urinary incontinence.

In a fourth preferred embodiment the disease or a disorder associated with the activity of potassium channels is epilepsy, seizures, absence seizures or convulsions.

In a fifth preferred embodiment the disease or a disorder associated with the activity of potassium channels is a respiratory disease, in particular asthma, cystic fibrosis, chronic obstructive pulmonary disease (COPD) or rhinorrhea.

The compounds tested all showed a biological activity in the micromolar and sub-micromolar range, i.e. of from below 1 to above 100 µM. Preferred compounds of the invention show a biological activity determined as described herein in the in the sub-micromolar and micromolar range, i.e. of from below 0.1 to about 10 µM.

### Pharmaceutical Compositions

In yet another aspect the invention provides novel pharmaceutical compositions comprising a therapeutically effective amount of the chemical compound of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers and/or diluents.

In a preferred embodiment, the invention provides pharmaceutical compositions comprising the chemical compound of the invention, or a pharmaceutically acceptable salt or derivative thereof, together with one or more pharmaceutically acceptable carriers therefor and, optionally, other therapeutic and/or prophylactic ingredients. The carrier(s) must be "acceptable" in the sense of being compatible with the other ingredients of the formulation and not deleterious to the recipient thereof.

Pharmaceutical compositions of the invention may be those suitable for oral, rectal, bronchial, nasal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like

For topical administration to the epidermis the chemical compound according to the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

A therapeutically effective dose refers to that amount of active ingredient which ameliorates the symptoms or condition. Therapeutic efficacy and toxicity, e.g. ED₅₀ and LD₅₀, may be determined by standard pharmacological procedures in cell cultures or experimental animals. The dose ratio between therapeutic and toxic effects is the therapeutic index and may be expressed by the ratio LD₅₀/ED₅₀. Pharmaceutical compositions which exhibit large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage depend on the nature and severity of the disease being treated and the route of administration, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### Methods of Therapy

In another aspect the invention provides the use of a compound of the invention for the manufacture of a medicament for the treatment or alleviation of a disease or a disorder or a condition of a living animal body, including a human, which disease, disorder or condition is responsive to modulation of potassium channels, in particular SK channels, and which method comprises comprising administering to such a living animal body, including a human, in need thereof a therapeutically-effective amount of a compound of the invention.

The preferred indications contemplated according to the invention are those stated above.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

A satisfactory result can, in certain instances, be obtained at a dosage as low as 0.005 mg/kg i.v. and 0.01 mg/kg p.o. The upper limit of the dosage range is about 10 mg/kg i.v. and 100 mg/kg p.o. Preferred ranges are from about 0.001 to about 1 mg/kg i.v. and from about 0.1 to about 10 mg/kg p.o.

### EXAMPLES

The invention is further illustrated with reference to the following examples.

### Example 1

### Preparatory Example

### Method A

### 6-Methyl-pyridine-2-carbonyl chloride(intermediate compound)

Thionylchloride (30 mL) was added to a heterogeneous solution of 6-methyl-picolinic acid (3.0 g, 21.8 mmol) in dichloromethane (30 mL) and the reaction mixture was heated to reflux for 2 hours. Excess thionylchloride was removed under reduced pressure to give 6-methyl-pyridine-2-carbonyl chloride (3.12 g, 20 mmol, 92%).

### 6-Methyl-pyridine-2-carboxylic acid (2-carbamoyl-phenyl)-amide

### (Intermediate compound)

A solution of 6-methyl-pyridine-2-carbonyl chloride (3.12 g, 20 mmol) in dichloromethane and *N*,*N*-dimethylformamide was added drop-wise to a solution of anthranilamide (2.74 g, 20.1 mmol) and triethylamine (5.6 mL, 40.2 mmol) in dichloromethane (30 mL). The mixture was stirred at room temperature for 4 hours and then concentrated under reduced pressure. The remaining residue was diluted with ethyl acetate (200 mL), washed with brine, dried over anhydrous sodium sulphate, filtered and evaporated. The crude product was purified by column chromatography to give 6-methyl-pyridine-2-carboxylic acid (2-carbamoyl-phenyl)-amide (4.8 g, 18.8 mmol, 94%).

### 2-(6-Methyl-pyridin-2-yl)-3-H-quinazolin-4-one (Intermediate compound)

6-Methyl-pyridine-2-carboxylic acid (2-carbamoyl-phenyl)-amide (4.8 g, 18.8 mmol) was added to a 1 M solution of potassium hydroxide (250 mL) and stirred at room temperature for 4 hours. The reaction mixture was extracted with ethyl acetate (3 x 100 mL) and the combined organic layers were dried over anhydrous sodium sulphate, filtered and evaporated. The crude product was purified by column chromatography to give 2-(6-methyl-pyridin-2-yl)-3H-quinazolin-4-one (3.25 g, 13.7 mmol, 73%).

### 4-Chloro-2-(6-methyl-pyridin-2-yl)-auinazoline (Intermediate compound)

Phosphorous oxychloride (1.7 mL, 11.4 mmol) was added drop-wise to a solution of 2-(6-methyl-pyridin-2-yl)-3H-quinazolin-4-one (3.25 g, 13.7 mmol) and *N*,*N-*dimethylaniline (2.64 mL, 20.5 mmol) in benzene (35 mL) and heated to reflux for 12 hours. The reaction mixture was quenched with ice, basified and extracted with ethyl acetate (3 x 100 ml). The combined organic layers were dried over anhydrous sodium sulphate, filtered and evaporated. The crude product was purified by column chromatography to give 4-chloro-2-(6-methyl-pyridin-2-yl)-quinazoline (2.1 g, 6.3 mmol, 55%).

### 4-Chloro-phenyl)-[2-(6-methyl-pyridin-2-yl)-quinazolin-4-yl]-amine (Compound A1)

A solution of 4-chloro-2-(6-methyl-pyridin-2-yl)-quinazoline (500 mg, 1.95 mmol), triethylamine (0.40 ml, 2.9 mmol) and 4-chloroaniline (300 mg, 2.34 mmol) in acetonitrile in a 10 mL vial was heated on sand bath at 70°C for 60 hours. The reaction mixture was concentrated under reduced pressure, loaded on a silica gel column and eluated with a mixture of ethyl acetate and hexane to give 4-chlorophenyl-[2-(6-methyl-pyridin-2-yl)-quinazolin-4-yl]-amine (450 mg, 66%). Mp. 256.1-259.1°C.

### (4-Chloro-phenyl)-[2-(6-fluoro-pyridin-2-yl)-quinazolin-4-yl]-amine (Compound A2)

The title compound was prepared according to Method A from 6-fluoropicolinic acid, anthranilamide and 4-chloroaniline. Mp. 118.3-121.4°C.

### [2-(6-Bromo-pyridin-2-yl)-guinazolin-4-yl]-(4-chloro-phenyl)-amine (Compound A3)

The title compound was prepared according to Method A from 6-bromopicolinic acid, anthranilamide and 4-chloroaniline. Mp. 242.4-247.9°C.

### (4-Chloro-phenyl)-[2-(6-methoxy-pyridin-2-yl)-quinazolin-4-yl]-amine (Reference Compound A4)

[2-(6-Bromo-pyridin-2-yl)-quinazolin-4-yl]-(4-chloro-phenyl)-amine (250 mg, 0.607 mmol) was dissolved in methanol (50 mL). Sodium methoxide (131 mg, 2.43 mmol) was added and heated to 60°C overnight. The reaction mixture was concentrated under reduced pressure. Water was added and the aquous phase was extracted with ethyl acetate. The combined organic layers were dried over sodium sulphate filtrated and evaporated in *vacuo.* The crude product was purified by column chromatography using chloroform containing 0.5% methanol as eluent. The product was crystallised from dichloromethane-hexane to give (4-chloro-phenyl)-[2-(6-methoxy-pyridin-2-yl)-quinazolin-4-yl]-amine (150 mg, 68%) as a yellow crystalline compound. Mp.156.8-160.0°C.

### 6-[4-(4-Chloro-phenylamino)-auinazolin-2-yl]-pyridin-2-ol (Reference Compound A5)

(4-Chloro-phenyl)-[2-(6-methoxy-pyridin-2-yl)-quinazolin-4-yl]-amine (200 mg, 0.551 mmol) was dissolved in tetrahydrofuran (100 mL). Hydrobromic acid (48%, 10 mL) was added slowly and the reaction mixture was heated at 60°C for 24 hours. The mixture was cooled down and basified with aqueous sodium hydrogencarbonate. The solid formed was filtered off, washed with water and dried in *vacuo.* This solid was titurated with a mixture of chloroform hexane to give 6-[4-(4-chloro-phenylamino)-quinazolin-2-yl]-pyridin-2-ol (133 mg, 46%) as a white solid. Mp > 350°C.

### Method B

### 3-Benzovl-pyridine-2-carboxylic acid (2-carbamoyl-phenyl)-amide (Intermediate compound)

1-Hydroxybenzoetriazole (25 mg, 0.18 mmol), N-(3-dimethylaminopropyl)-N'-ethylcarbodiimide, hydrochloride (520 mg, 2.7 mmol) and triethylamine (360 mg, 3.6 mmol) were added to a solution of 3-benzoyl-pyridine-2-carbonylic acid (500 mg, 2 mmol) in dichloromethane (50 mL) under a nitrogen atmosphere at 0°C. After stirring for 30 minutes at 0°C anthranilamide (250 mg, 1.8 mmol) was added and stirring was continued at room temperature for 12 hours. The reaction mixture was concentrated under reduced pressure and diluted with ethyl acetate (250 mL), washed with brine, dried over anhydrous sodium sulphate, filtered and evaporated. The crude product was purified by column chromatography to give 3-benzoyl-pyridine-2-carboxylic acid (2-carbamoyl-phenyl)-amide (50 mg, 0.14 mmol, 80%).

### 2-(3-Benzoyl-pyridin-2-yl)-3-H-quinazolin-4-one (Intermediate compound)

3-Benzoyl-pyridine-2-carboxylic acid (2-carbamoyl-phenyl)-amide (3.0 g, 8.7 mmol) was added to a 1 M solution of potassium hydroxide (60 mL) and stirred at room temperature for 1 hour. The reaction mixture was extracted with ethyl acetate (3 x 100 mL), and the combined organic layers were dried over anhydrous sodium sulphate, filtered and evaporated. The crude product was purified by column chromatography to give 2-(3-benzoyl-pyridin-2-yl)-3-H-quinazolin-4-one (1.6 g, 4.8 mmol, 55%).

### [2-(4-Chloro-guinazolin-2-yl)-pyridin-3-yl]-phenyl-methanone (Intermediate compound)

Phosphorous oxychloride (600 mg, 3.9 mmol) was added drop-wise to a solution of 2-(3-benzoyl-pyridin-2-yl)-3-H-quinazolin-4-one (1.6 g, 4.8 mmol) and N, N-dimethylaniline (650 mg, 6.6 mmol) in benzene (35 mL) and heated to reflux for 12 hours. The reaction mixture was quenched with ice, basified using 10% aquous sodium hydrogencarbonate and extracted with ethyl acetate (3 x 100 ml). The combined organic layers were dried over anhydrous sodium sulphate, filtered and evaporated. The crude product was purified by column chromatography to give [2-(4-chloro-quinazolin-2-yl)-pyridin-3-yl]-phenyl-methanone (750 mg, 2.2 mmol, 45%).

### [2-(4-Cyclohexylamino-quinazolin-2-yl)-pyridin-3-yl]-phenyl-methanone (Compound B1)

A solution of [2-(4-chloro-quinazolin-2-yl)-pyridin-3-yl]-phenyl-methanone (120 mg, 0.34 mmol), triethylamine (0.4 ml, 2.9 mmol) and cyclohexylamine (0.38 ml, 0.34 mmol) in acetonitrile in a 10 mL vial was heated on sand bath at 70°C for 60 hours. The reaction mixture was concentrated under reduced pressure, loaded on a silica gel and eluated with a mixture of ethyl acetate/heptane to give [2-(4-cyclohexylamino-quinazolin-2-yl)-pyridin-3-yl]-phenyl-methanone (44 mg, 0.11 mmol, 32%). Mp. 204-208°C.

### Cyclohexyl-[2-(6-methyl-pyridin-2-yl)-auinazolin-4-yl]-amine (Compound B2)

The title compound was prepared according to Method A from 6-methylpicolinic acid, anthranilamide and cyclohexylamine. Mp. 253.1-254.9°C.

### (4-Chloro-benzvl)-[2-(6-methyl-pyridin-2-yl)-quinazolin-4-yl]-amine (Compound B3)

The title compound was prepared according to Method A from 6-methylpicolinic acid, anthranilamide and 6-chlorobenzylamine. Mp. 209.9-212.2°C.

### [2-(6-Chloro-pyridin-2-yl)-quinazolin-4-yl]-cyclohexyl-amine (Compound B4)

The title compound was prepared according to Method B from 6-chloropicolinic acid, anthranilamide and cyclohexylamine. Mp. 218.4-220.9°C.

### (4-Chloro-phenyl)-[2-(6-chloro-pyridin-2-yl)-auinazolin-4-yl]-amine (Compound B5)

The title compound was prepared according to Method B from 6-chloropicolinic acid, anthranilamide and 4-chloroaniline. Mp. 211.9-213.2°C.

### (4-Chloro-benzyl)-[2-(6-chloro-pyridin-2-yl)-guinazolin-4-yl]-amine (Compound B6)

The title compound was prepared according to Method B from 6-chloropicolinic acid, anthranilamide and 4-chlorobenzylamine. Mp. 187.4-189.7°C.

### {2-[4-(4-Chloro-phenylamino)-quinazolin-2-yl-pyridin-3-yl}-Dhenyl-methanone (Compound B7)

The title compound was prepared according to Method B from 3-benzoyl pyridine-2-carboxylic acid, anthranilamide and 4-chloroaniline. Mp. 189.5-192.4°C.

### {2-[4-(4-Chloro-benzylamino)-quinazolin-2-yl]-pyridin-3-yl}-phenyl-methanone (Compound B8)

The title compound was prepared according to Method B from 3-benzoyl pyridine-2-carboxylic acid, anthranilamide and 4-chlorobenzylamine. Mp. 147.1-149.4°C.

### Cyclohexyl-(2-isoquinolin-1-yl-guinazolin-4-yl)-amine (Compound B9)

Was prepared according to Method B from isoquinoline-l-carboxylic acid, anthranilamide and cyclohexylamine. Mp. 274.8-276.9°C.

### (4-Chloro-phenyl)-(2-isoauinolin-1-yl-auinazolin-4-yl)-amine (Compound B10)

The title compound was prepared according to Method B from isoquinoline-1-carboxylic acid, anthranilamide and 4-chloroaniline. Mp. 279-280.5°C.

### (4-Chloro-benzyl)-(2-isoquinolin-1-yl-quinazolin-4-yl)-amine (Compound B11)

The title compound was prepared according to Method B from isoquinoline-1-carboxylic acid, anthranilamide and 4-chlorobenzylamine. Mp. 221.4-224.5°C.

### Example 2

### Biological Activity

This example demonstrates the biological activity of a compound representative of the invention (Compound A1).

The ionic current through small-conductance Ca²+-activated K⁺ channels (SK channels, subtype 3) is recorded using HEK293 tissue culture cells expressing hSK3 channels in a whole-cell configuration of the patch-clamp technique as described in e.g. WO 2006/100212.

For activators a SC₁₀₀ value may be estimated. The SC₁₀₀ value is defined as the Stimulating Concentration required for increasing the baseline current by 100%. The SC₁₀₀ value determined for Compound A1 of the invention was well below 0.1 µM, which is an indication of its strong SK3 activating properties.

## Claims

1. A pyridinyl-quinazoline derivative of Formula I 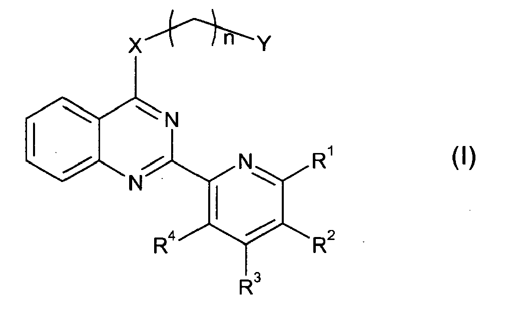 an enantiomer or a mixture of its enantiomers, an N-oxide thereof, or a pharmaceutically acceptable salt thereof, wherein
n is 0, 1, 2 or 3;
X represents O, S or NR'; wherein
R' represents hydrogen, alkyl, cycloalkyl or cycloalkyl-alkyl;
or, when n is 0 and X is NR', R' together with Y and together with the nitrogen to which they are attached form a heterocyclic ring, which heterocyclic ring may optionally be substituted with alkyl of phenyl;
Y represents alkyl, cycloalkyl, alkyl-cycloalkyl, alkenyl or phenyl, which phenyl is optionally substituted one or more times with substituents selected from the group consisting of alkyl, amino-alkyl, alkyl-amino, alkyl-amino-alkyl, hydroxy-alkyl, alkoxy-alkyl, cycloalkyl, cycloalkyl-alkyl, alkenyl, halo, haloalkyl, hydroxy, alkoxy, haloalkoxy, cyano, nitro and amino;
or, when n is 0, Y together with R' and together with the nitrogen to which they are attached form a heterocyclic ring, which heterocyclic ring may optionally be substituted with alkyl of phenyl; and
R¹ represents methyl, ethyl, propyl, fluoro, chloro or bromo; and R², R³ and R⁴ represent hydrogen, or
R¹, R² and R³ represent hydrogen; and R⁴ represent benzoyl, or
R¹ and R², or R² and R³, or R³ and R⁴, respectively, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and
the remaining of R¹, R², R³ and R⁴ represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl.

2. The pyridinyl-quinazoline derivative of claim 1, wherein n is 0, 1, 2 or 3.

3. The pyridinyl-quinazoline derivative of either one of claims 1-2, wherein
X represents O, S or NR'; wherein
R' represents hydrogen, alkyl, cycloalkyl or cycloalkyl-alkyl;
or, when n is 0 and X is NR', R' together with Y and together with the nitrogen to which they are attached form a heterocyclic ring, which heterocyclic ring may optionally be substituted with alkyl of phenyl.

4. The pyridinyl-quinazoline derivative of any one of claims 1-3, wherein
Y represents alkyl, cycloalkyl, alkyl-cycloalkyl, alkenyl or phenyl, which phenyl is optionally substituted one or more times with substituents selected from the group consisting of alkyl, amino-alkyl, alkyl-amino, alkyl-amino-alkyl, hydroxy-alkyl, alkoxy-alkyl, cycloalkyl, cycloalkyl-alkyl, alkenyl, halo, haloalkyl, hydroxy, alkoxy, haloalkoxy, cyano, nitro and amino;
or, when n is 0, Y together with R' and together with the nitrogen to which they are attached form a heterocyclic ring, which heterocyclic ring may optionally be substituted with alkyl of phenyl.

5. The pyridinyl-quinazoline derivative of any one of claims 1-4, wherein
R¹ represents methyl, ethyl, propyl, fluoro, chloro or bromo; and R², R³ and R⁴ represent hydrogen, or
R¹, R² and R³ represent hydrogen; and R⁴ represent benzoyl, or
R¹ and R², or R² and R³, or R³ and R⁴, respectively, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and
the remaining of R¹, R², R³ and R⁴ represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl.

6. The pyridinyl-quinazoline derivative of claim 5, wherein
R¹ and R², independently of each other, represent hydrogen, alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl; and
R³ and R⁴, together form a benzo-fused carbocyclic ring, optionally substituted with alkyl, cycloalkyl, cycloalkyl-alkyl, alkenyl, halo, haloalkyl, hydroxy, alkoxy, haloalkoxy, alkoxy-carbonyl, cyano, nitro, amino, phenyl or benzoyl.

7. The pyridinyl-quinazoline derivative of claim 1, wherein
n is 0 or 1;
X represents NH;
Y represents cycloalkyl or phenyl, which phenyl is optionally substituted with alkyl or halo; and
R¹ represents methyl, ethyl, propyl, fluoro, chloro or bromo; and R², R³ and R⁴ represent hydrogen, or
R¹, R² and R³ represent hydrogen; and R⁴ represent benzoyl; or
R¹ and R² represent hydrogen; and
R³ and R⁴, together form a benzo-fused carbocyclic ring.

8. The pyridinyl-quinazoline derivative of claim 7, which is
(4-Chloro-phenyl)-[2-(6-methyl-pyridin-2-yl)-quinazolin-4-yl]-amine;
[2-(4-Cyclohexylamino-quinazolin-2-yl)-pyridin-3-yl]-phenyl-methanone;
Cyclohexyl-[2-(6-methyl-pyridin-2-yl)-quinazolin-4-yl]-amine;
(4-Chloro-benzyl)-[2-(6-methyl-pyridin-2-yl)-quinazolin-4-yl]-amine;
[2-(6-Chloro-pyridin-2-yl)-quinazolin-4-yl]-cyclohexyl-amine;
(4-Chloro-phenyl)-[2-(6-chloro-pyridin-2-yl)-quinazolin-4-yl]-amine;
(4-Chloro-benzyl)-[2-(6-chloro-pyridin-2-yl)-quinazolin-4-yl]-amine;
{2-[4-(4-Chloro-phenylamino)-quinazolin-2-yl]-pyridin-3-yl}-phenyl-methanone;
{2-[4-(4-Chloro-benzylamino)-quinazolin-2-yl]-pyridin-3-yl}-phenyl-methanone;
Cyclohexyl-(2-isoquinolin-1-yl-quinazolin-4-yl)-amine;
(4-Chloro-phenyl)-(2-isoquinolin-1-yl-quinazolin-4-yl)-amine;
(4-Chloro-benzyl)-(2-isoquinolin-1-yl-quinazolin-4-yl)-amine;
(4-Chloro-phenyl)-[2-(6-fluoro-pyridin-2-yl)-quinazolin-4-yl]-amine; or
[2-(6-Bromo-pyridin-2-yl)-quinazolin-4-yl]-(4-chloro-phenyl)-amine;
or a pharmaceutically acceptable salt thereof.

9. A pharmaceutical composition comprising a therapeutically-effective amount of a pyridinyl-quinazoline derivative according to any of claims 1-8, or a pharmaceutically-acceptable addition salt thereof, together with at least one pharmaceutically-acceptable carrier or diluent.

10. The use of a pyridinyl-quinazoline derivative according to any of claims 1-8 for the manufacture of a medicament for the treatment, prevention or alleviation of a disease or a disorder or a condition of a mammal, including a human, which disease, disorder or condition is associated with the activity of potassium channels.

11. The use according to claim 10, wherein the disease or a disorder associated with the activity of potassium channels is a respiratory disease, epilepsy, convulsions, seizures, absence seizures, vascular spasms, coronary artery spasms, renal disorders, polycystic kidney disease, bladder spasms, urinary incontinence, bladder outflow obstruction, erectile dysfunction, gastrointestinal dysfunction, secretory diarrhoea, ischaemia, cerebral ischaemia, ischaemic heart disease, angina pectoris, coronary heart disease, ataxia, traumatic brain injury, Parkinson's disease, bipolar disorder, psychosis, schizophrenia, anxiety, depression, mood disorders, dementia, memory and attention deficits, Alzheimer's disease, amyotrophic lateral sclerosis (ALS), dysmenorrhea, narcolepsy, Reynaud's disease, intermittent claudication, Sjorgren's syndrome, arrhythmia, hypertension, myotonic muscle dystrophia, spasticity, xerostomi, diabetes type II, hyperinsulinemia, premature labour, baldness, cancer, irritable bowel syndrome, immune suppression, migraine or pain.

12. The use according to claim 10, wherein the disease or a disorder associated with the activity of potassium channels is a respiratory disease, urinary incontinence, erectile dysfunction, anxiety, epilepsy, psychosis, schizophrenia, amyotrophic lateral sclerosis (ALS) or pain.

13. The use according to claim 10, wherein the activity of potassium channels is a respiratory disease, in particular asthma, cystic fibrosis, chronic obstructive pulmonary disease (COPD) or rhinorrhea.

14. A pyridinyl-quinazoline derivative according to any of claims 1-8, any of its enantiomers or any mixture of its enantiomers, or a pharmaceutically acceptable salt thereof, for use as a medicament.

15. A pyridinyl-quinazoline derivative according to any of claims 1-8, any of its enantiomers or any mixture of its enantiomers, or a pharmaceutically acceptable salt thereof, for use in the treatment, prevention or alleviation of a disease or a disorder or a condition of a mammal, including a human, which disease, disorder or condition is associated with the activity of potassium channels.

## Patentansprüche

1. Pyridinyl-chinazolin-Derivat der Formel I 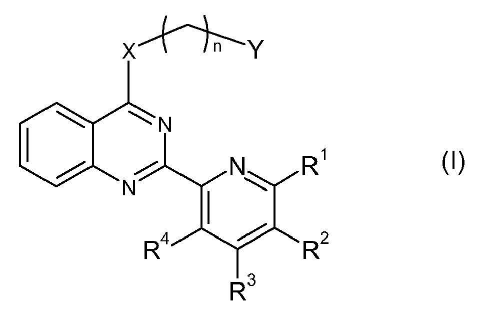 ein Enantiomer oder eine Mischung von seinen Enantiomeren, ein N-Oxid davon oder ein pharmazeutisch verträgliches Salz davon, worin
n 0, 1, 2 oder 3 ist;
X O, S oder NR' bedeutet; worin
R' Wasserstoff, Alkyl, Cycloalkyl oder Cycloalkylalkyl bedeutet;
oder, wenn n 0 ist und X NR' ist, R' zusammen mit Y und zusammen mit dem Stickstoff, an den sie gebunden sind, einen heterocyclischen Ring bilden, wobei dieser heterocyclische Ring gegebenenfalls mit Alkyl oder Phenyl substituiert sein kann;
Y Alkyl, Cycloalkyl, Alkylcycloalkyl, Alkenyl oder Phenyl bedeutet, wobei dieses Phenyl gegebenenfalls einfach oder mehrfach mit Substituenten substituiert ist, die ausgewählt sind aus der Gruppe bestehend aus Alkyl, Aminoalkyl, Alkylamino, Alkylaminoalkyl, Hydroxyalkyl, Alkoxyalkyl, Cycloalkyl, Cycloalkylalkyl, Alkenyl, Halogen, Halogenalkyl, Hydroxy, Alkoxy, Halogenalkoxy, Cyano, Nitro und Amino;
oder, wenn n 0 ist, Y zusammen mit R' und zusammen mit dem Stickstoff, an den sie gebunden sind, einen heterocyclischen Ring bilden, wobei dieser heterocyclische Ring gegebenenfalls mit Alkyl oder Phenyl substituiert sein kann; und
R¹ Methyl, Ethyl, Propyl, Fluor, Chlor oder Brom bedeutet; und R², R³ und R⁴ Wasserstoff bedeuten, oder
R¹, R² und R³ Wasserstoff bedeuten; und R⁴ Benzoyl bedeutet, oder
R¹ und R², oder R² und R³, oder R³ und R⁴, jeweils zusammen einen Benzo-kondensierten carbocyclischen Ring bilden, der gegebenenfalls substituiert ist mit Alkyl, Cycloalkyl, Cycloalkylalkyl, Alkenyl, Halogen, Halogenalkyl, Hydroxy, Alkoxy, Halogenalkoxy, Alkoxycarbonyl, Cyano, Nitro, Amino, Phenyl oder Benzoyl; und
die übrigen von R¹, R², R³ und R⁴ Wasserstoff, Alkyl, Cycloalkyl, Cycloalkylalkyl, Alkenyl, Halogen, Halogenalkyl, Hydroxy, Alkoxy, Halogenalkoxy, Alkoxycarbonyl, Cyano, Nitro, Amino, Phenyl oder Benzoyl bedeuten.

2. Pyridinyl-chinazolin-Derivat nach Anspruch 1, worin n 0, 1, 2 oder 3 ist.

3. Pyridinyl-chinazolin-Derivat nach einem der Ansprüche 1-2, worin,
X O, S oder NR' bedeutet; worin
R' Wasserstoff, Alkyl, Cycloalkyl oder Cycloalkylalkyl bedeutet;
oder, wenn n 0 ist und X NR' ist, R' zusammen mit Y und zusammen mit dem Stickstoff, an den sie gebunden sind, einen heterocyclischen Ring bilden, wobei dieser heterocyclische Ring gegebenenfalls mit Alkyl oder Phenyl substituiert sein kann.

4. Pyridinyl-chinazolin-Derivat nach einem der Ansprüche 1-3, worin
Y Alkyl, Cycloalkyl, Alkylcycloalkyl, Alkenyl oder Phenyl bedeutet, wobei dieses Phenyl gegebenenfalls einfach oder mehrfach mit Substituenten substituiert ist, die ausgewählt sind aus der Gruppe bestehend aus Alkyl, Aminoalkyl, Alkylamino, Alkylaminoalkyl, Hydroxyalkyl, Alkoxyalkyl, Cycloalkyl, Cycloalkylalkyl, Alkenyl, Halogen, Halogenalkyl, Hydroxy, Alkoxy, Halogenalkoxy, Cyano, Nitro und Amino;
oder, wenn n 0 ist, Y zusammen mit R' und zusammen mit dem Stickstoff, an den sie gebunden sind, einen heterocyclischen Ring bilden, wobei dieser heterocyclische Ring gegebenenfalls mit Alkyl oder Phenyl substituiert sein kann.

5. Pyridinyl-chinazolin-Derivat nach einem der Ansprüche 1-4, worin
R¹ Methyl, Ethyl, Propyl, Fluor, Chlor oder Brom bedeutet; und R², R³ und R⁴ Wasserstoff bedeuten, oder
R¹, R² und R³ Wasserstoff bedeuten; und R⁴ Benzoyl bedeutet, oder
R¹ und R², oder R² und R³, oder R³ und R⁴, jeweils zusammen einen Benzo-kondensierten carbocyclischen Ring bilden, der gegebenenfalls substituiert ist mit Alkyl, Cycloalkyl, Cycloalkylalkyl, Alkenyl, Halogen, Halogenalkyl, Hydroxy, Alkoxy, Halogenalkoxy, Alkoxycarbonyl, Cyano, Nitro, Amino, Phenyl oder Benzoyl; und
die übrigen von R¹, R², R³ und R⁴ Wasserstoff, Alkyl, Cycloalkyl, Cycloalkylalkyl, AIkenyl, Halogen, Halogenalkyl, Hydroxy, Alkoxy, Halogenalkoxy, Alkoxycarbonyl, Cyano, Nitro, Amino, Phenyl oder Benzoyl bedeuten.

6. Pyridinyl-chinazolin-Derivat nach Anspruch 5, worin
R¹ und R² unabhängig voneinander Wasserstoff, Alkyl, Cycloalkyl, Cycloalkylalkyl, Alkenyl, Halogen, Halogenalkyl, Hydroxy, Alkoxy, Halogenalkoxy, Alkoxycarbonyl, Cyano, Nitro, Amino, Phenyl oder Benzoyl bedeuten; und
R³ und R⁴ zusammen einen Benzo-kondensierten carbocyclischen Ring bilden, der gegebenenfalls substituiert ist mit Alkyl, Cycloalkyl, Cycloalkylalkyl, Alkenyl, Halogen, Halogenalkyl, Hydroxy, Alkoxy, Halogenalkoxy, Alkoxycarbonyl, Cyano, Nitro, Amino, Phenyl oder Benzoyl.

7. Pyridinyl-chinazolin-Derivat nach Anspruch 1, worin
n 0 oder 1 ist;
X NH bedeutet;
Y Cycloalkyl oder Phenyl bedeutet, wobei dieses Phenyl gegebenenfalls mit Alkyl oder Halogen substituiert ist; und
R¹ Methyl, Ethyl, Propyl, Fluor, Chlor oder Brom bedeutet; und R², R³ und R⁴ Wasserstoff bedeuten, oder
R¹, R² und R³ Wasserstoff bedeuten; und R⁴ Benzoyl bedeutet; oder
R¹ und R² Wasserstoff bedeuten; und
R³ und R⁴ zusammen einen Benzo-kondensierten carbocyclischen Ring bilden.

8. Pyridinyl-chinazolin-Derivat nach Anspruch 7, welches
(4-Chlor-phenyl)-[2-(6-methyl-pyridin-2-yl)-chinazolin-4-yl]-amin;
[2-(4-Cyclohexylamino-chinazolin-2-yl)-pyridin-3-yl]-phenyl-methanon;
Cyclohexyl-[2-(6-methyl-pyridin-2-yl)-chinazolin-4-yl]-amin;
(4-Chlor-benzyl)-[2-(6-methyl-pyridin-2-yl)-chinazolin-4-yl]-amin;
[2-(6-Chlor-pyridin-2-yl)-chinazolin-4-yl]-cyclohexyl-amin;
(4-Chlor-phenyl)-[2-(6-chlor-pyridin-2-yl)-chinazolin-4-yl]-amin;
(4-Chlor-benzyl)-[2-(6-chlor-pyridin-2-yl)-chinazolin-4-yl]-amin;
{2-[4-(4-Chlor-phenylamino)-chinazolin-2-yl]-pyridin-3-yl}-phenyl-methanon;
{2-[4-(4-Chlor-benzylamino)-chinazolin-2-yl]-pyridin-3-yl}-phenyl-methanon;
Cyclohexyl-(2-isochinolin-1-yl-chinazolin-4-yl)-amin;
(4-Chlor-phenyl)-(2-isochinolin-1-yl-chinazolin-4-yl)-amin;
(4-Chlor-benzyl)-(2-isochinolin-1-yl-chinazolin-4-yl)-amin;
(4-Chlor-phenyl)-[2-(6-fluor-pyridin-2-yl)-chinazolin-4-yl]-amin; oder
[2-(6-Brom-pyridin-2-yl)-chinazolin-4-yl]-(4-chlor-phenyl)-amin ist; oder ein pharmazeutisch verträgliches Salz davon.

9. Pharmazeutische Zusammensetzung umfassend eine therapeutisch wirksame Menge von einem Pyridinyl-chinazolin-Derivat nach einem der Ansprüche 1-8, oder einem pharmazeutisch verträglichen Additionssalz davon, zusammen mit wenigstens einem pharmazeutisch verträglichen Träger oder Verdünnungsmittel.

10. Verwendung eines Pyridinyl-chinazolin-Derivats nach einem der Ansprüche 1-8 für die Herstellung eines Medikaments für die Behandlung, Verhütung oder Linderung einer Krankheit oder einer Störung oder eines Zustands eines Säugers, einschließlich eines Menschen, wobei diese Krankheit, diese Störung oder dieser Zustand mit der Aktivität von Kaliumkanälen zusammenhängt.

11. Verwendung nach Anspruch 10, wobei es sich bei der Krankheit oder einer Störung, die mit der Aktivität von Kaliumkanälen zusammenhängt, um eine Atemwegserkrankung, Epilepsie, Krämpfe, Anfälle, Petit-mal-Epilepsie, Gefäßspasmen, Koronararterienspasmen, Nierenerkrankungen, polyzystische Nierenerkrankung, Blasenspasmen, Harninkontinenz, Blasenauslassobstruktion, erektile Dysfunktion, gastrointestinale Dysfunktion, sekretorische Diarrhoe, Ischämie, zerebrale Ischämie, ischämische Herzkrankheit, Angina pectoris, koronare Herzkrankheit, Ataxie, traumatische Hirnverletzung, die Parkinson-Krankheit, eine bipolare Störung, Psychose, Schizophrenie, Angst, Depression, affektive Psychosen, Demenz, Gedächtnisdefizite und Aufmerksamkeitsdefizite, die Alzheimer-Krankheit, amyotrophe Lateralsklerose (ALS), Dysmenorrhoe, Narkolepsie, die Reynaud-Krankheit, intermittierendes Hinken, das Sjögren-Syndrom, Arrhythmie, Hochdruck, myotone Muskeldystrophie, Spastizität, Xerostomie, Diabetes Typ II, Hyperinsulinämie, eine Frühgeburt, Kahlheit, Krebs, das Reizdarmsyndrom, eine Immunsuppression, Migräne oder Schmerzen handelt.

12. Verwendung nach Anspruch 10, wobei es sich bei der Krankheit oder einer Störung, die mit der Aktivität von Kaliumkanälen zusammenhängt, um eine Atemwegserkrankung, Harninkontinenz, erektile Dysfunktion, Angst, Epilepsie, Psychose, Schizophrenie, amyotrophe Lateralsklerose (ALS) oder Schmerzen handelt.

13. Verwendung nach Anspruch 10, wobei es sich bei der Krankheit oder einer Störung, die mit der Aktivität von Kaliumkanälen zusammenhängt, um eine Atemwegserkrankung, insbesondere Asthma, zystische Fibrose, chronisch obstruktive Lungenerkrankung (COPD) oder Rhinorrhoe handelt.

14. Pyridinyl-chinazolin-Derivat nach einem der Ansprüche 1-8, beliebige von seinen Enantiomeren oder eine beliebige Mischung von seinen Enantiomeren, oder ein pharmazeutisch verträgliches Salz davon, zur Verwendung als ein Medikament.

15. Pyridinyl-chinazolin-Derivat nach einem der Ansprüche 1-8, beliebige von seinen Enantiomeren oder eine beliebige Mischung von seinen Enantiomeren, oder ein pharmazeutisch verträgliches Salz davon, zur Verwendung bei der Behandlung, Verhütung oder Linderung einer Krankheit oder einer Störung oder eines Zustands eines Säugers, einschließlich eines Menschen, wobei diese Krankheit, diese Störung oder dieser Zustand mit der Aktivität von Kaliumkanälen zusammenhängt.

## Revendications

1. Dérivé de pyridinyl-quinazoline de formule I 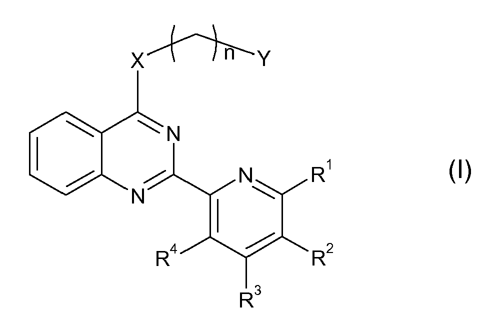 un énantiomère ou un mélange de ses énantiomères, un N-oxyde de celui-ci, ou un sel pharmaceutiquement acceptable de celui-ci, dans lequel
n est 0, 1, 2 ou 3 ;
X représente O, S ou NR' ; où
R' représente un hydrogène, un alkyle, un cycloalkyle ou un cycloalkyl-alkyle ;
ou, lorsque n est 0 et X est NR', R' conjointement avec Y et conjointement avec l'azote auquel ils sont attachés forment un noyau hétérocyclique, lequel noyau hétérocyclique peut être facultativement substitué par un alkyle de phényle ;
Y représente un alkyle, un cycloalkyle, un alkyl-cycloalkyle, un alcényle ou un phényle, lequel phényle est facultativement substitué une ou plusieurs fois par des substituants choisis dans le groupe constitué par un alkyle, un amino-alkyle, un alkyl-amino, un alkyl-amino-alkyle, un hydroxy-alkyle, un alcoxy-alkyle, un cycloalkyle, un cycloalkyl-alkyle, un alcényle, un halogéno, un halogénoalkyle, un hydroxy, un alcoxy, un halogénoalcoxy, un cyano, un nitro et un amino ;
ou, lorsque n est 0, Y conjointement avec R' et conjointement avec l'azote auquel ils sont attachés forment un noyau hétérocyclique, lequel noyau hétérocyclique peut être facultativement substitué par un alkyle de phényle ; et
R¹ représente un méthyle, un éthyle, un propyle, un fluoro, un chloro ou un bromo ; et R² R³ et R⁴ représentent un hydrogène, ou
R¹, R² et R³ représentent un hydrogène ; et R⁴ représente un benzoyle, ou
R¹ et R², ou R² et R³, ou R³ et R⁴, respectivement, forment ensemble un noyau carbocyclique benzo-condensé, facultativement substitué par un alkyle, un cycloalkyle, un cycloalkyl-alkyle, un alcényle, un halogéno, un halogénoalkyle, un hydroxy, un alcoxy, un halogénoalcoxy, un alcoxy-carbonyle, un cyano, un nitro, un amino, un phényle ou un benzoyle ; et
le reste de R¹, R², R³ et R⁴ représente un hydrogène, un alkyle, un cycloalkyle, un cycloalkyl-alkyle, un alcényle, un halogéno, un halogénoalkyle, un hydroxy, un alcoxy, un halogénoalcoxy, un alcoxy-carbonyle, un cyano, un nitro, un amino, un phényle ou un benzoyle.

2. Dérivé de pyridinyl-quinazoline selon la revendication 1, dans lequel n est 0, 1, 2 ou 3.

3. Dérivé de pyridinyl-quinazoline selon l'une quelconque des revendications 1-2, dans lequel
X représente O, S ou NR' ; où
R' représente un hydrogène, un alkyle, un cycloalkyle ou un cycloalkyl-alkyle ;
ou, lorsque n est 0 et X est NR', R' conjointement avec Y et conjointement avec l'azote auquel ils sont attachés forment un noyau hétérocyclique, lequel noyau hétérocyclique peut être facultativement substitué par un alkyle de phényle.

4. Dérivé de pyridinyl-quinazoline selon l'une quelconque des revendications 1 à 3, dans lequel
Y représente un alkyle, un cycloalkyle, un alkyl-cycloalkyle, un alcényle ou un phényle, lequel phényle est facultativement substitué une ou plusieurs fois par des substituants choisis dans le groupe constitué par un alkyle, un amino-alkyle, un alkyl-amino, un alkyl-amino-alkyle, un hydroxy-alkyle, un alcoxy-alkyle, un cycloalkyle, un cycloalkyl-alkyle, un alcényle, un halogéno, un halogénoalkyle, un hydroxy, un alcoxy, un halogénoalcoxy, un cyano, un nitro et un amino ;
ou, lorsque n est 0, Y conjointement avec R' et conjointement avec l'azote auquel ils sont attachés forment un noyau hétérocyclique, lequel noyau hétérocyclique peut être facultativement substitué par un alkyle de phényle.

5. Dérivé de pyridinyl-quinazoline selon l'une quelconque des revendications 1 à 4, dans lequel
R¹ représente un méthyle, un éthyle, un propyle, un fluoro, un chloro ou un bromo ; et R², R³ et R⁴ représentent un hydrogène, ou
R¹, R² et R³ représentent un hydrogène ; et R⁴ représente un benzoyle, ou
R¹ et R², ou R² et R³, ou R³ et R⁴, respectivement, forment ensemble un noyau carbocyclique benzo-condensé, facultativement substitué par un alkyle, un cycloalkyle, un cycloalkyl-alkyle, un alcényle, un halogéno, un halogénoalkyle, un hydroxy, un alcoxy, un halogénoalcoxy, un alcoxy-carbonyle, un cyano, un nitro, un amino, un phényle ou un benzoyle ; et
le reste de R¹, R², R³ et R⁴ représente un hydrogène, un alkyle, un cycloalkyle, un cycloalkyl-alkyle, un alcényle, un halogéno, un halogénoalkyle, un hydroxy, un alcoxy, un halogénoalcoxy, un alcoxy-carbonyle, un cyano, un nitro, un amino, un phényle ou un benzoyle.

6. Dérivé de pyridinyl-quinazoline selon la revendication 5, dans lequel
R¹ et R², indépendamment l'un de l'autre, représentent un hydrogène, un alkyle, un cycloalkyle, un cycloalkyl-alkyle, un alcényle, un halogéno, un halogénoalkyle, un hydroxy, un alcoxy, un halogénoalcoxy, un alcoxy-carbonyle, un cyano, un nitro, un amino, un phényle ou un benzoyle ; et
R³ et R⁴ forment ensemble un noyau carbocyclique benzo-condensé, facultativement substitué par un alkyle, un cycloalkyle, un cycloalkyl-alkyle, un alcényle, un halogéno, un halogénoalkyle, un hydroxy, un alcoxy, un halogénoalcoxy, un alcoxy-carbonyle, un cyano, un nitro, un amino, un phényle ou un benzoyle.

7. Dérivé de pyridinyl-quinazoline selon la revendication 1, dans lequel
n est 0 ou 1 ;
X représente NH ;
Y représente un cycloalkyle ou un phényle, lequel phényle est facultativement substitué par un alkyle ou un halogéno ; et
R¹ représente un méthyle, un éthyle, un propyle, un fluoro, un chloro ou un bromo ; et R², R³ et R⁴ représentent un hydrogène, ou
R¹, R² et R³ représentent un hydrogène ; et R⁴ représente un benzoyle ; ou
R¹ et R² représentent un hydrogène ; et
R³ et R⁴ forment ensemble un noyau carbocyclique benzo-condensé.

8. Dérivé de pyridinyl-quinazoline selon la revendication 7, qui est
la (4-chloro-phényl)-[2-(6-méthyl-pyridin-2-yl)-quinazolin-4-yl]amine ;
la [2-(4-cyclohexylamino-quinazolin-2-yl)-pyridin-3-yl]-phényl-méthanone ;
la cyclohexyl-[2-(6-méthyl-pyridin-2-yl)-quinazolin-4-yl]-amine ;
la (4-chloro-benzyl)-[2-(6-méthyl-pyridin-2-yl)-quinazolin-4-yl]-amine ;
la [2-(6-chloro-pyridin-2-yl)-quinazolin-4-yl]-cyclohexyl-amine ;
la (4-chloro-phényl)-[2-(6-chloro-pyridin-2-yl)-quinazolin-4-yl]amine ;
la (4-chloro-benzyl)-[2-(6-chloro-pyridin-2-yl)-quinazolin-4-yl]amine ;
la {2-[4-(4-chloro-phénylamino)-quinazolin-2-yl]-pyridin-3-yl}-phényl-méthanone ;
la {2-[4-(4-chloro-benzylamino)-quinazolin-2-yl]-pyridin-3-yl}-phényl-méthanone ;
la cyclohexyl-(2-isoquinoléin-1-yl-quinazolin-4-yl)-amine;
la (4-chloro-phényl)-(2-isoquinoléin-1-yl-quinazolin-4-yl)-amine ;
la (4-chloro-benzyl)-(2-isoquinoléin-1-yl-quinazolin-4-yl)-amine ;
la (4-chloro-phényl)-[2-(6-fluoro-pyridin-2-yl)-quinazolin-4-yl]-amine ; ou
la [2-(6-bromo-pyridin-2-yl)-quinazolin-4-yl]-(4-chloro-phényl)-amine ;
ou un sel pharmaceutiquement acceptable de celui-ci.

9. Composition pharmaceutique comprenant une quantité thérapeutiquement efficace d'un dérivé de pyridinyl-quinazoline selon l'une quelconque des revendications 1 à 8, ou d'un sel d'addition pharmaceutiquement acceptable de celui-ci, conjointement avec au moins un véhicule ou diluant pharmaceutiquement acceptable.

10. Utilisation d'un dérivé de pyridinyl-quinazoline selon l'une quelconque des revendications 1 à 8, pour la fabrication d'un médicament pour le traitement, la prévention ou l'atténuation d'une maladie ou d'un trouble ou d'une affection d'un mammifère, incluant un être humain, laquelle maladie, lequel trouble ou laquelle affection est associé(e) à l'activité des canaux potassiques.

11. Utilisation selon la revendication 10, dans laquelle la maladie ou un trouble associé(e) à l'activité des canaux potassiques est une maladie respiratoire, une épilepsie, des convulsions, des crises d'épilepsie, des absences, des spasmes vasculaires, des spasmes des artères coronaires, des troubles rénaux, une maladie polykystique des reins, des spasmes de la vessie, une incontinence urinaire, une obstruction de l'écoulement de la vessie, une dysérection, un dysfonctionnement gastro-intestinal, une diarrhée sécrétoire, une ischémie, une ischémie cérébrale, une cardiopathie ischémique, une angine de poitrine, une maladie coronarienne, une ataxie, une lésion cérébrale traumatique, une maladie de Parkinson, un trouble bipolaire, une psychose, une schizophrénie, une anxiété, une dépression, des troubles de l'humeur, une démence, des déficiences de la mémoire et de l'attention, une maladie d'Alzheimer, une sclérose latérale amyotrophique (SLA), une dysménorrhée, une narcolepsie, une maladie de Reynaud, une claudication intermittente, un syndrome de Sjorgren, une arythmie, une hypertension, une dystrophie musculaire myotonique, une spasticité, une xérostomie, un diabète de type II, une hyperinsulinémie, un travail prématuré, une calvitie, un cancer, un syndrome du côlon irritable, une suppression immunitaire, une migraine ou une douleur.

12. Utilisation selon la revendication 10, dans laquelle la maladie ou un trouble associé(e) à l'activité des canaux potassiques est une maladie respiratoire, une incontinence urinaire, une dysérection, une anxiété, une épilepsie, une psychose, une schizophrénie, une sclérose latérale amyotrophique (SLA) ou une douleur.

13. Utilisation selon la revendication 10, dans laquelle l'activité des canaux potassiques est une maladie respiratoire, en particulier un asthme, une mucoviscidose, une bronchopneumopathie obstructive chronique (BPCO) ou une rhinorrhée.

14. Dérivé de pyridinyl-quinazoline selon l'une quelconque des revendications 1 à 8, l'un quelconque de ses énantiomères ou tout mélange de ses énantiomères, ou un sel pharmaceutiquement acceptable de celui-ci, pour une utilisation en tant que médicament.

15. Dérivé de pyridinyl-quinazoline selon l'une quelconque des revendications 1 à 8, l'un quelconque de ses énantiomères ou tout mélange de ses énantiomères, ou un sel pharmaceutiquement acceptable de celui-ci, pour une utilisation dans le traitement, la prévention ou l'atténuation d'une maladie ou d'un trouble ou d'une affection d'un mammifère, incluant un être humain, laquelle maladie, lequel trouble ou laquelle affection est associé(e) à l'activité des canaux potassiques.
